# NEUE EUROPÄISCHE PATENTSCHRIFT

(11) **EP 0 679 187 B2**
(45) Veröffentlichungstag und Bekanntmachung des Hinweises auf die Entscheidung über den Einspruch: **04.07.2001**
(45) Hinweis auf die Patenterteilung: 21.05.1997
(21) Anmeldenummer: 94904568.6
(22) Anmeldetag: 12.01.1994
(51) Int. Cl.: C12N 15/49, C07K 14/00, C12P 21/06, A61K 39/21, A61K 38/00, C12P 21/08, G01N 33/569, G01N 33/68

(54) **VERFAHREN ZUR GEWINNUNG NATIVER, OLIGOMERER, GLYKOSYLIERTER EKTODOMÄNEN VIRALER MEMBRANPROTEINE, DEREN VERWENDUNG, INSBESONDERE ALS IMPFSTOFF GEGEN HIV**
PROCESS FOR RECOVERING NATIVE, OLIGOMERIC, GLYCOSYLATED ECTODOMAINS OF VIRAL MEMBRANE PROTEINS, THEIR USE, IN PARTICULAR AS VACCINE AGAINST HIV
PROCEDE PERMETTANT D'OBTENIR DES ECTODOMAINES NATIFS, OLIGOMERIQUES, GLYCOSYLES DE PROTEINES VIRALES MEMBRANAIRES, LEUR UTILISATION, NOTAMMENT COMME VACCIN CONTRE LE VIH

(30) Priorität: 16.01.1993 DE 4301017
(43) Veröffentlichungstag der Anmeldung: 02.11.1995
(73) Patentinhaber: SCHAWALLER, Manfred, D-75173 Pforzheim (DE)
(72) Erfinder: SCHAWALLER, Manfred, D-75173 Pforzheim (DE)
(74) Vertreter: Müller-Boré & Partner Patentanwälte
(86) Internationale Anmeldenummer: DE9400022
(87) Internationale Veröffentlichungsnummer: WO9416081

(56) Entgegenhaltungen:
- EP-A- 0 335 635
- WO-A-89/02461
- WO-A-91/13906
- WO-A-92/19742
- VIROLOGY 172 (1). 1989. 367-369. SCHAWALLER, M. ET AL 'STUDIES WITH CROSSLINKING REAGENTS ON THE OLIGOMERIC STRUCTURE OF THE ENV GLYCOPROTEIN OF HIV.' in der Anmeldung erwähnt
- JOURNAL OF VIROLOGY Bd. 65, Nr. 5 , Mai 1991 , BALTIMORE, US Seiten 2718 - 2723 HORAL, P. ET AL. 'Continuous epitopes of the human immunodeficiency virus type 1 (HIV-1) transmembrane glycoprotein and reactivity of human sera to synthetic peptides representing various HIV-1 isolates' in der Anmeldung erwähnt
- J VIROL 67 (6). 1993. 3601-3604. MORIKAWA, Y. ET AL 'LEGITIMATE AND ILLEGITIMATE CLEAVAGE OF HUMAN IMMUNODEFICIENCY VIRUS GLYCOPROTEINS BY FURIN.'
- Owens et al., Virology (1990) 179:287
- Earl et al., P.N.A.S. (1990) 87:648
- Pinter et al., J. Virol. (1989) 63:2674
- Thompson et al., J. Virol. (1988) 62:4653
- Formanowski et al., Virus Research (1988) 10:177
- Steimer et al., Biotechnology Therapeutics (1990-1991) 2:63
- Navarro et al., Virology (1993) 192:234
- Wengler et al., Virology (1999) 257:472
- 12th World Aids Conference- Geneva, 28/06/1998: AIDS VACCINE RESEARCH. A TOP PRIORITY FOR PASTEUR MERIEUX CONNAUGHT (RONE-POULENC GROUP)

## Beschreibung

Die vorliegende Erfindung betrifft ein Verfahren zur Gewinnung von isolierten Ektodomänen viraler Membranproteine in nativer, oligomerer und glykosylierter Form, insbesondere von Ektodomänen des Oberflächenproteins gp160 des menschlichen Immunschwächevirus HIV (Human Immunodeficiency Virus), dem Erreger von AIDS (Aquired Immune Deficiency Syndrome), weiterhin die durch dieses Verfahren gewonnenen Ektodomänen selbst sowie deren Verwendung als Impfstoff, insbesondere die Verwendung nativer Ektodomänen des Oberflächenproteins gp160 des menschlichen Immunschwächevirus HIV als Impfstoff gegen HIV. Bestandteil dieses Verfahrens zur Gewinnung von isolierten Ektodomänen viraler Membranproteine ist auch ein Verfahren zur Ermittlung geeigneter Stellen für den Einbau von Erkennungssequenzen in die zu gewinnende Proteindomänen, die als enzymatische Spaltstellen dienen, insbesondere als Collagenasespaltstellen, sowie Erkennungssequenzen für Collagenasen selbst.

Ein probates Mittel zur Prophylaxe von Viruserkrankungen ist die Immunisierung, die Impfung des Körpers gegen die Viren. Durch Impfung konnten viele in früheren Zeiten oft epidernieartig verlaufende Viruserkrankungen wie z.B. die Pocken und Polio eingedämmt werden. Zur Erlangung einer solchen aktiven Immunisierung gegen ein Virus setzt man den Körper, genauer gesagt das Immunsystem, einem viralen Antigen aus. Dies kann geschehen beispielsweise durch Injektion von inaktivierten bzw. attenuierten Viren oder auch von Teilen, z.B. von Proteinen des Virus. Grundsätzlich setzt sich das Immunsystem des Menschen aus zwei verschiedenen Teilsystemen zusammen. Die zelluläre Immunantwort (T-Zellen) und die humorale Immunantwort (B-Zellen, Antikörper). Bei der Neuinfektion mit einem Virus stellt die humorale Immunantwort, durch B-Zellen vermittelt, den wichtigsten Abwehrmechanismus dar.
Das Immunsystem des Körpers reagiert auf das Antigen mit der Bildung von spezifischen Antikörpern, die das Antigen und damit das Virus erkennen und binden und dadurch dessen Inaktivierung in die Wege leiten. Darüberhinaus werden sogenannte "Gedächtniszellen" gebildet, das sind spezielle Lymphozyten, die bei einer späteren Infektion mit "ihrem" Virus bzw. "ihrem" Antigen aktiviert werden und das Immunsystem sehr rasch zur Synthese von großen Mengen der antigenspezifischen Antikörper stimulieren. Dadurch kann das Immunsystem wesentlich schneller auf einen Virusbefall reagieren, als wenn es vorher noch nicht mit dem entsprechenden Antigen konfrontiert worden ist.

Die Verwendung von attenuierten Viren zur Impfung kann allerdings insofern problematisch sein, als nicht ausgeschlossen werden kann, daß die verwendeten Viren im Körper wieder virulent werden und es zum Ausbruch der Viruserkrankung kommt. Aus diesem Grund ist es vorzuziehen, ein isoliertes Protein des Virus als Immunogen zu verwenden. Hierzu verwendet man am besten ein Oberflächenprotein des Virus, da dieses normalerweise der humoralen Immunantwort gut zugänglich ist. Idealerweise verwendet man nur einen Teil eines Oberflächenproteins, und zwar denjenigen Teil, der sich außen auf der Vrusoberfläche befindet, die sogenannte Ektodomäne, da beim intakten Virus nur dieser Teil für Antikörper zugänglich ist. Bei der Herstellung einer Proteindomäne, die zur Impfung gegen ein Virus eingesetzt werden soll, ist allerdings zu beachten, daß sie möglichst die native Form haben sollte, da anderenfalls die Gefahr besteht, daß Antikörper gebildet werden, die das native Protein, und damit das Virus nicht erkennen und somit bei der Neutralisation wirkungslos sind. Solche Moleküle oder Molekülteile, die aufgrund Ihrer dreidimensionalen Struktur von Antikörpern erkannt werden, nennt man konformationelle bzw. strukturelle Epitope, diejenigen, die an ihrer Aminosäuresequenz erkannt werden, sequentielle Epitope. Es zeigt sich immer mehr, daß es nur sehr wenige sequentielle Epitope gibt, die meisten antigenen Epitope sind strukturell, d.h. sie müssen die korrekte dreidimensionale Faltung der Polypeptidkette aufweisen, um von entsprechenden Antikörpem erkannt zu werden. Nativ bedeutet in diesem Zusammenhang, daß die räumliche Struktur der verwendeten Proteindomäne identisch oder nahezu identisch ist mit der Struktur des entsprechenden, natürlicherweise auf oder in dem Virus vorkommenden Proteinbereiches. Dies schließt gegebenenfalls das Vorhandensein von Oligomerie und / oder Glykosylierung der Proteindomäne ein. Ein wichtiges Kriterium hierfür ist, daß Antikörper, die gegen eine isolierte native, virale Proteindomäne gebildet worden sind, auch den Virus bzw. das entsprechende native Protein im Virus erkennen und binden.
Große Probleme bereitet heutzutage das menschliche Immunschwächevirus HIV ein Retrovirus. Seit es im Jahre 1984 als Ursache von AIDS identifiziert worden ist, werden große Anstrengungen unternommen, auch gegen dieses Virus einen Impfstoff herzustellen. In jüngerer Zeit ist es gelungen, Affen mit abgeschwächten SIV-Viren (Simian Immunodeficiency Vrus), nahen Verwandten des HIV, erfolgreich zu immunisieren, was vermuten läßt, daß auch Menschen erfolgreich gegen HIV immunisiert werden könnten mittels abgeschwächten HIV. Die Problematik, die abgeschwächte Viren als Impfstoffe mit sich bringen, wurde schon angesprochen. Bei einer tödlich verlaufenden Krankheit wie AIDS kann dieses Risiko, daß der abgeschwächte Virus wieder virulent wird, nicht in Kauf genommen werden, auch wenn es sehr klein ist. Siehe hierzu auch: Koff, W.C. und Hoth, D.F. in der Zeitschrift "Science", **241,** 426-432 (1988).
Aus diesem Grund konzentriert sich ein großer Teil der Forschung über HIV-Impfstoffe darauf, einzelne Proteine, insbesondere das Oberflächenprotein des Virus, das sogenannte gp160, auch env-Glykoprotein genannt, oder Fragmente davon als Antigen zu verwenden. [Eine allgemeine Übersicht gibt der Artikel "AIDS-Impfstoffe" von T.H. Matthews et al. in der Zeitschrift "Spektrum der Wissenschaft" 12, 134-142 (1988)].
Das env-Glykoprotein von HIV, dessen Aminosäuresequenz bekannt ist [Ratner et al., "Nature" **313,** 277-284 (1985)] wird in der Wirtszelle zunächst als stark glykosyliertes Vorläuferprotein gp160 synthetisiert und anschließend im Golgi-Apparat zu den beiden Domänen gp120 und gp41 prozessiert. Die gp120-Domäne, die sich außen auf der Virusmembran befindet, ist für die Bindung an den zellulären Rezeptor CD4 verantwortlich und sowohl gp120 als auch das Vorläuferprotein gp160 binden mit hoher Affinität an diesen CD4-Rezeptor. Gp41, das eine Transmembrandomäne aufweist, ist beteiligt an der Fusion von viraler und zellulärer Membran während des Eindringens des Virus in die Zelle, wahrscheinlich durch Interaktion eines "Fusionspeptids", das am Aminoterminus von gp41 liegt, mit der Lipiddoppelschicht der Wirtszellmembran.

Vielversprechend erscheint der Ansatz, für die Entwicklung eines HIV-Impfstoffs lediglich den äußeren Teil des env-Glykoproteins zu verwenden. Dem liegt die bereits erwähnte Überlegung zugrunde, daß der nach außen exponierte Teil des Glykoproteins am leichtesten der humoralen Immunantwort zugänglich ist und daher eine Immunisierung gegen diese Ektodomäne am erfolgversprechendsten ist.

Die Gewinnung der Ektodomäne des HIV-Oberflächenproteins kann auf unterschiedliche Art und Weise erfolgen. Sie kann beispielsweise durch mechanische, chemische oder enzymatische Methoden von der Virushülle "abgeschnitten" werden. Dabei ist die Gefahr groß, daß das Protein denaturiert wird und zur Bildung von Antikörpern führt, die das native Oberflächenprotein und damit den Virus nur schlecht oder gar nicht erkennen. Robey et al. ["Proc. Natl. Acad. Sci. USA", **83,** 7023-7027 (1986)] gewinnen gp120 beispielsweise durch Ablösen des Proteins von der Oberfläche HIV-infizierter Zellen mittels des recht starken Detergenz Triton X100. Auch wenn die Autoren ihre Präparation "natives gp120" nennen, ist in Anbetracht des verwendeten Detergenz und der verwendeten Reinigungsmethoden zu bezweifeln, daß das so gewonnene gp120 tatsächlich in nativer und oligomerer Form vorliegt.

In der europäischen Patentanmeldung EP 0 328 403 ist vorgeschlagen, lediglich Peptide aus HIV-gp120 mit einer Länge zwischen 28 und 33 Aminosäuren als Impfstoff gegen HIV zu verwenden. Diese Peptide liegen aber nicht in nativer, insbesondere oligomerer Form vor.

Ein gentechnologischer Ansatz besteht darin, ein Stopcodon quasi als "Sollbruchstelle" knapp vor Beginn des gp41-Gens einzubauen. Bei der Expression des Gens in geeigneten Zellen bestimmt die Lage des Stopcodons dann die Lage des carboxyterminalen Endes der Ektodomäne des Oberflächenproteins, das heißt, es wird nur der äußere Teil des Oberflächenproteins, die Domäne gp120 synthetisiert. Auf diese Art synthetisiert, liegt gp120 aber als Monomer vor, was nicht den natürlichen Verhältnissen am Virus entspricht, da das env-Glykoprotein wie die meisten viralen Glykoproteine in nativer Form als Oligomer vorliegt. Häufig sind diese Proteine Trimere, für das HIV-1 env-Glykoprotein konnte kürzlich gezeigt werden, daß es in nativer Form ein Tetramer ist [Schawaller M. et al., "Virology", **172**, 367-369 (1989)]. Dies erklärt, weshalb monomeres gp120 nicht zu geeigneten Antikörpern führt und somit als Impfstoff wenig brauchbar ist. Auch eigene Experimente des Erfinders, bei denen gp120 von Zellen synthetisiert wurde, die mit einem recombinanten Vaccinia-Virus, durch den env exprimiert wird, infiziert worden waren, zeigten, daß gp120 nicht stabil in oligomerer Form gebildet wird. Die Versuchsergebnisse deuten darauf hin, daß gp41 während der Biosynthese des env-Glykoproteins anwesend sein muß, um die Oligomerisierung zu gewährleisten. Wahrscheinlich liegt diejenige Domäne von gp41, die für diese Oligomerisierung verantwortlich ist, innerhalb der 129 aminoterminalen Aminosäuren von gp41 [Earl, P.L. et al. "Proc. Natl. Acad. Sci. USA", **87**, 648-652 (1990)].

Da gp41 offensichtlich eine wichtige Bedeutung bei der Synthese von nativem gp160 hat, wurden auch Versuche unternommen, das komplette gp160 in stabiler Form herzustellen. Barret et al. ["AIDS Research and Human Retroviruses" **5** (2), 159-170 (1989)] gewinnen gp160 beispielsweise durch Expression des env-Gens in eukaryontischen Zellen und anschließender Reinigung, wobei sie allerdings neben Detergentien auch das stark chaotrop wirkende Kaliumthiocyanat verwenden, welches Proteine denaturiert. Eigene Arbeiten des Autors haben gezeigt, daß die Reindarstellung von gp160 große Schwierigkeiten bereitet, und zwar aus folgenden Gründen:
Gp160 ist, wie oben beschrieben, ein Membranprotein. Zur Herauslösung dieses Proteins aus der Membran sind Detergentien notwendig. Diese sind auch deshalb nötig, da isolierte gp160 Moleküle bei Abwesenheit von Detergentien aggregieren. Detergentien führen aber zu einer Veränderung des Moleküls. Es wurde festgesteltt, daß gp160 mit Detergentien eine Veränderung des Proteaseverdaumusters im Vergleich zum unbehandelten Protein zeigt. Beim Entfernen des Detergenz aus der Lösung, was spätestens dann nötig wird, wenn das Protein beispielsweise zum Zwecke der Impfung einem Patienten injiziert werden soll, zerfällt das Oligomer zum Monomer Aus den genannten Gründen erscheinen die Chancen, reines gp160 in nativer Form herstellen zu können, sehr gering zu sein.

Der wesentliche Nachteil, den alle bisherigen Ansätze zur Entwicklung eines Impfstoffes gegen HIV auf der Basis der isolierten Ektodomäne des env-Glykoproteins gemeinsam haben, ist also höchstwahrscheinlich der, daß das als Immunogen verwendete Protein nicht in nativer und oligomerer Form vorhanden war. Dies wird dadurch deutlich, daß die von den Autoren angegebenen Titer, die mittels ELISA ermittelt wurden, sehr hoch (um 1/100 000), die im Neutralisierungstest gemessenen Titer dagegen extrem niedrig sind (oft weit weniger als 1/1000). Dies deutet darauf hin, daß die jeweils verwendeten Antigene zwar starke immunogene Wirkung besitzen, also eine große Menge Antikörper dagegen gebildet werden, daß diese Antikörper aber das native Protein, und damit auch den Virus kaum oder gar nicht erkennen.

Die niedrigen Titer im Neutralisierungstest zeigen, daß das native Antigen erkennende Antikörper nur in sehr niedrigen Titem vorhanden sind. Niedertitrige Antikörper aber führen häufig zu einer Erhöhung der Virusinfektivität [Prabhakar et al., Nature **290**, 590 (1981)]. Mit anderen Worten, anstatt den Wirt vor der Infektion zu schützen, wird durch virusspezifische Immunglobuline die Infektivität erhöht. In diesem Fall hat die Vaccine das Gegenteil des gewünschten Effektes zur Folge. Die geschilderte Problematik ergibt sich auch bei allen anderen Viren, die eine Oberflächenmembran mit membrangebundenen Proteinen besitzen.

Neben der Verwendung als Impfstoff werden native Proteine bzw. Proteindomänen auch als Immuntherapeutikum oder für diagnostische Zwecke verwendet. Für die Diagnose werden sowohl Antigene, als auch die dagegen gerichteten Antikörper eingesetzt.

Aufgabe der vorliegenden Erfindung ist es, ein Verfahren zur Gewinnung von isolierten Ektodomänen viraler Membranproteine, insbesondere Ektodomänen des env-Glykoproteins gp160 des menschlichen Immunschwächevirus HIV zur Verfügung zu stellen, die beispielsweise als Impfstoff, zur Immuntherapie oder für diagnostische Zwecke verwendet werden können. Diese Aufgabe umfaßt auch die Zurverfügungstellung eines Verfahrens zur Identifikation einer geeigneten Stelle zum Einbau einer Erkennungssequenz für proteinspaltende Enzyme, insbesondere Collagenasen, in virale Membranproteine, beispielsweise in das HIV-env Glykoprotein gp160, sowie die Zurverfügungstellung einer geeigneten Collagenasespaltstelle.

Diese Aufgabe wird durch Bereitstellung eines Verfahrens zur Gewinnung von isolierten Ektodomänen viraler Membranproteine in nativer, oligomerer und glykosylierter Form gelöst, **dadurch gekennzeichnet, daß**
- in das Gen, das für ein virales, die gewünschte Ektodomäne enthaltendes Membranprotein kodiert, eine Nukleotidsequenz insertiert wird, die für eine Aminosäuresequenz kodiert, welche eine Erkennungssequenz für proteinspaltende Enzyme darstellt,
- die so erhaltene Gen-Mutante in eukaryontischen Zellen zum Erhalt des Membranproteins in nativer, oligomerer und glykosylierter Form einschließlich der insertierten Erkennungssequenz exprimiert wird,
- die Ektodomäne in nativer, oligomerer und glykosylierter Form durch enzymatische Spaltung des Membranproteins mit für die Erkennungssequenz spezifischen proteinspaltenden Enzymen von den eukaryontischen Zellen oder Teilen hiervon abgetrennt wird, und
- die Ektodomäne in nativer, oligomerer und glykosylierter Form aus dem Gemisch isoliert wird.

Ein weiterer Gegenstand der vorliegenden Erfindung sind isolierte Ektodomänen viraler Membranproteine in nativer, oligomerer und glykosylierter Form, wobei das korrespondierende Gen des viralen Membranproteins derart manipuliert ist, daß das virale Membranprotein eine zusätzliche Aminosäuresequenz beinhaltet, die als Erkennungssequenz für proteinspaltende Enzyme oder für Glykolipidanker dient, wobei die isolierten Ektodomänen aus der Domäne gp120 des HIV env-Glykoproteins, dem 131 bis 165 Aminosäuren langen aminoterminalen Stück der Domäne gp41 des HIV env-Glykoproteins sowie gegebenenfalls dem nach der enzymatischen Spältung verbleibenden Stück der Erkennungssequenz zusammengesetzt sind, wobei die für die Erkennungssequenz kodierende Nukleotidsequenz in einen Bereich des für das HIV env-Glykoprotein kodierenden Gens insertiert wird, der bei dem daraus exprimierten HIV env-Glykoprotein dem Bereich entspricht, der begrenzt wird durch die erste und fünfzigste Aminosäure vor Beginn des transmembranen Bereichs des HIV env-Glykoproteins.

Ganz allgemein gesprochen bedeutet dies, daß zunächst an geeigneter Stelle in das Gen, das für die Ausgangsform (z.B. gp160), das Vorläuferprotein der zu gewinnenden Proteindomäne, codiert, eine für eine Aminosäuresequenz codierende Nukleotidsequenz eingebaut wird, welche eine Erkennungssequenz für proteinspaltende Enzyme, insbesondere Proteasen, oder Glykolipidanker darstellt. Nach erfolgter Synthese des Proteins durch geeignete eukaryontische Zellen wird es an der insertierten Erkennungssequenz mit den entsprechenden Enzymen gespalten und anschließend die gewünschte Proteindomäne aufgereinigt. Bei Verwendung des HIV env-Glykoproteins gp160 als Vorläuferprotein resultieren aus diesem Verfahren oligomere, insbesondere tetramere Glykoproteine, deren Monomere eine elektrophoretische Mobilität von ca. 140 kD aufweisen und die im folgenden gp140 genannt werden.

Der Erfindung liegt also die Idee zugrunde, zunächst das komplette Protein zu synthetisieren, da es nur auf diese Weise in nativer und oligomerer Form synthetisierbar ist, und nach erfolgter Synthese die gewünschte Proteindomäne an spezifischer Stelle enzymatisch abzuspalten. Zuvor wird das korrespondierende Gen derart manipuliert, daß das fertige Protein eine zusätzliche Aminosäuresequenz beinhaltet, die als Erkennungssequenz für proteinspaltende Enzyme oder für Glycolipidanker dient. Diese zusätzliche Sequenz ändert nicht die native Proteinstruktur. Durch enzymatische Spaltung des Gesamtproteins an dieser Erkennungssequenz erhält man die gewünschte Proteindomäne in nativer und oligomerer Struktur. Ihrer Natur nach ist diese Erkennungssequenz eine Aminosäuresequenz, die von Proteasen erkannt und gespalten wird, es kann aber auch eine Erkennungssequenz für Lipidanker sein, wobei mittels Lipasen ebenfalls eine gezielte Spaltung möglich ist [siehe hierzu: Ferguson, M.A.J.; An. Rev. Biochem. **57**, 285-320 (1988)]

Im ersten Schritt des erfindungsgemäßen Verfahrens muß zunächst der für den Einbau einer Erkennungssequenz geeignete Bereich im Protein identifiziert werden. Dies erfolgt mittels einer "Pepscan" genannten Methode. Dann wird in der dem identifizierten Proteinbereich entsprechenden Gensequenz eine Erkennungssequenz für eine Endonuklease, z.B. EcoR I erzeugt, in die hinein dann die Sequenz, die für die gewünschte Aminosäuresequenz - die Erkennungssequenz im Protein - codiert, eingebaut wird. Es kann anstatt EcoR I auch jede andere Endonuklease verwendet werden, es sollten allerdings in der näheren Umgebung der Erkennungssequenz dieser Endonuklease keine weiteren gleichartigen Erkennungssequenzen vorhanden sein, da ansonsten die Klonierungseffizienz abnehmen kann. Das so erhaltene Gen wird in einen Vektor, beispielsweise ein Plasmid, eingebaut, der in geeignete eukaryontische Zellen transfiziert wird, welche dann das gesamte Protein inclusive der insertierten Erkennungssequenz synthetisieren. Auf diese Weise erhält man das gesamte Protein in nativer, oligomerer und glycosilierter Form. Daraufhin wird mit einem geeigneten Enzym, das das Protein an der eingebauten Erkennungssequenz schneidet, die gewünschte Domäne vom Rest des Proteins abgetrennt. Mittels geeigneter Reinigungsmethoden wie z.B. Affinitätschromatographie, lonenaustauschchromatographie oder Gradientenzentrifugation wird die gewünschte Proteindomäne anschließend aufgereinigt. Was das verwendete spaltende Enzym betrifft, so ist das wesentliche Kriterium für dessen Eignung, daß es die Spaltstelle an deren Aminosäuresequenz erkennt. Diese Bedingung erfüllen bakterielle Collagenasen, auch vereinzelt pflanzliche Collagenasen sowie z.B. der Blutgerinnungsfaktor Xa.

In einer bevorzugten Ausführungsform der Erfindung wird zur Erhöhung der Ausbeute die natürliche Prozessierungssequenz des Vorläuferproteins zerstört. Dies erfolgt beispielsweise durch Austausch einer oder mehrerer Aminosäuren in der Prozessierungssequenz. Dies hat zur Folge, daß das Vorläuferprotein, im Falle von HIV das gp160, nicht in die einzelnen Domänen (bei HIV also gp41 und gp120) aufgespalten wird.
Das Vorläuferprotein wird im rauhen endoplasmatischen Retikulum (RER) der Zelle synthetisiert. Erst im Golgi-Apparat, also während des Transports zur Zelloberfläche, wird das Protein prozessiert, d.h. in die einzelnen Domänen aufgespalten. Wird die Prozessierungssequenz des Vorläuferproteins zerstört, gewinnt man nicht nur den Anteil an Vorläuferprotein, der zum Zeitpunkt der Membranpräparation noch im RER vorhanden ist, sondern zusätzlich auch das bereits zur Zelloberfläche transportierte unprozessierte Vorläuferprotein.

In einer weiteren bevorzugten Ausführungsform der Erfindung erfolgt die Einschleusung des mutanten Gens in die eukaryontische Zelle nicht mittels eines Plasmids, sondern mittels eines rekombinanten Virus, insbesondere mittels eines rekombinanten Vaccinia Virus. Besonders für die Herstellung größerer Mengen der gewünschten viralen Proteindomänen ist die Verwendung eines rekombinanten Vaccinia Virus als Vektor vorteilhaft. Die jeweilige Genmutante kann beispielsweise in das Thymidinkinase-Gen des Vaccinia Virus insertiert werden. Da die Wahrscheinlichkeit für eine solche Rekombination bei ca 1/1000 bis 1/10 000 liegt, ist es vorteilhaft, nach der Rekombination auf den rekombinaten Virus zu selektionieren. Dies kann beispielsweise durch Einsatz des Mutagens Bromodesoxyuridin in HTK⁻-Zellen erfolgen. Nur der Wildtyp-Vaccinia Virus (TK⁺) baut dieses mutagene Nukleotidderivat in die DNA ein und schon nach zweimaliger Selektion liegt der Anteil der rekombinanten Viren (TK⁻) bei ca. 90%. Hohe Proteinausbeuten erhält man bei Verwendung eines Late-Promotors, beispielsweise des Cowpox-160K-Promotors, der ein starker Vaccinia-Promotor ist [Patel, D. D. et al., "Proc. Natl. Acad. Sci. USA" 85, 9431-9435 (1988)]. Eine Übersicht über die Verwendung des Vaccinia Virus als Vektor für die Expression von Genen im Cytoplasma eukaryotischer Zellen gibt Moss, B., ["Science" **252**, 1662-1667 (1988)].

Weitere Spezifikationen im Hinblick auf das erfindungsgemäße Verfahren, eine erfindungsgemäße Erkennungssequenz sowie die erfindungsgemäßen nativen Proteindomänen und deren Verwendung sind Gegenstand der Unteranspruche.

Die Erfindung, die oben ganz allgemein skizziert ist, wurde gemacht bei der Untersuchung der Struktur des env-Glykoproteins von HIV, sie kann aber prinzipiell bei allen Viren angewandt werden, die eine Envelope-Membran besitzen. Für Kristallisationsversuche mit der Ektodomäne des env-Glykoproteins war es nötig, diese Ektodomäne in der nativen oligomeren Form ohne die hydrophobe transmembrane Domäne herzustellen. Der Oligomerisierungsprozeß von viralen Glykoproteinen vom Typ I ist aber wahrscheinlich abhängig von der Anwesenheit dieser hydrophoben transmembranen Domäne während der Biosynthese. Darüberhinaus scheint die Faltung der Polypeptidkette dadurch beeinflußt zu werden, daß die Polypeptidkette während der Synthese membrangebunden ist. Um die Ektodomäne des env-Glykoproteins in nativer Form zu erhalten, wurde nach dem Einbau einer für eine Collagenasespaltstelle codierenden Sequenz in das env-Gen zunächst das gesamte, mutante env-Glykoprotein in COS-Zellen synthetisiert, danach die gewünschte Ektodomäne mittels einer bakteriellen Collagenase vom synthetisierten Gesamtprotein abgespalten und anschließend aufgereinigt.

Als großes Problem erwies sich dabei, eine geeignete Stelle im Protein für den Einbau der Collagenasespaltstelle - der Erkennungssequenz - zu identifizieren. Es hat sich nämlich gezeigt, daß es nur einen sehr kleinen Bereich im Protein gibt, an der die Erkennungssequenz eingebaut und das Protein dann auch erfolgreich gespalten werden kann. Bei rein empirischer Ermittlung einer solchen geeigneten Stelle mußten mehr als 30 verschiedene env-Gen-Mutanten hergestellt werden, bei denen an unterschiedlichen Stellen in der gewünschten Region von gp41 die Erkennungssequenz eingebaut wurde, um eine Stelle zu finden, an der die gewünschte proteolytische Spaltung möglich war.

Die im folgenden verwendete Numerierung der Aminosäuren des HIV env-Glykoproteins bzw. der Basen des HIV env-Gens bezieht sich auf die von Ratner verwendete Numerierung [Ratner et al., "Nature" **313**, 277-284 (1985)].

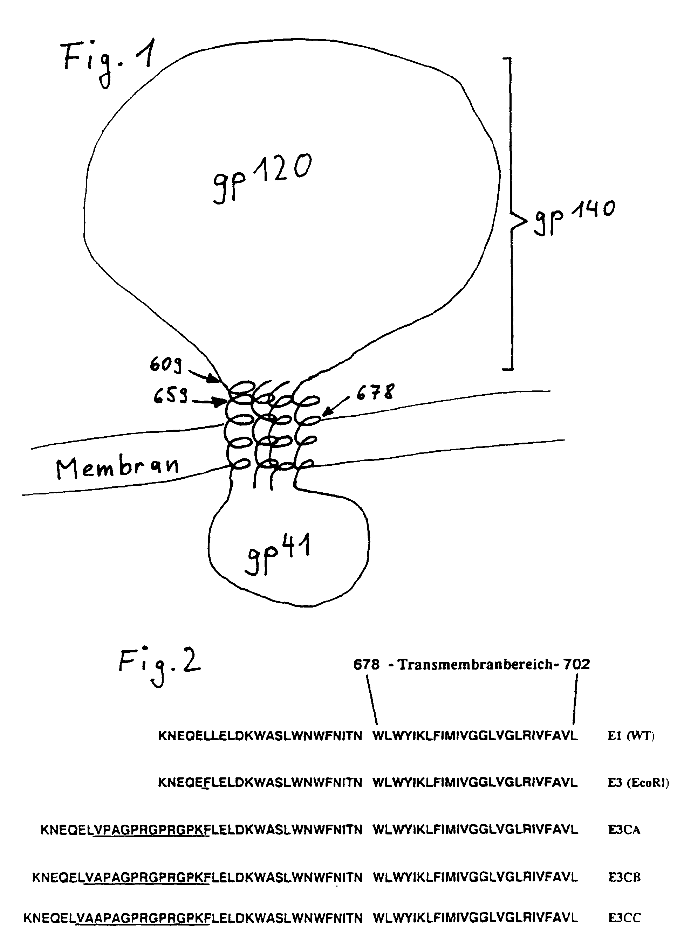

Die genaue räumliche Struktur des env-Glykoproteins ist nicht bekannt, die Struktur des Bereichs des Proteins, in dem die Proteasespaltstelle sinnvollerweise eingebaut werden kann, stellt sich folgendermaßen dar (siehe auch Fig. 1):
Geht man von Hydrophobizitätsprofilen von gp41 aus, ist die erste Aminosäure des transmembranen Bereichs nach Ansicht des Erfinders Trp 678. Über die genaue Lage des transmembranen Bereichs von gp41 ist sich die Fachwelt allerdings nicht einig. Bolognesi, P.D. ["Mol. Biol. Med." 7, 1-15 (1990)] nimmt beispielsweise an, daß der transmembrane Bereich des gp160 von Aminosäure 691 bis Aminosäure 712 reicht. Klar ist, daß die vier konservierten Glykosylierungsstellen von gp41, die an den Positionen 611, 616, 624 und 637 lokalisiert sind, aminoterminal zur transmembranen Domäne, also in der Ektodomäne liegen müssen. Weiterhin kann man aus UV-Circulardichroismus-Messungen an gereinigten gp120- und gp160-Präparationen auf die Sekundärstruktur von gp41 schließen. Diese Messungen haben ergeben, daß gp120 fast ausschließlich aus β-Faltblattstrukturen besteht, während gp160 ca. 30% α-helicale Bereiche aufweist. Dies deutet darauf hin, daß die Sekundärstruktur von gp41 im wesentlichen eine α-Helix ist. Darüberhinaus kann man aus Sequenzanalysen des env-Gens verschiedener HIV-Stämme schließen, daß die Region in gp41 zwischen Pro 609 und dem Transmembranbereich (Trp 678) ebenfalls α-helicale Struktur besitzt. Diese Annahme wird noch dadurch unterstützt, daß keiner der untersuchten HIV-Stämme zwischen Pro 609 und Trp 678 ein Prolin besitzt, welches wahrscheinlich die α-helicale Struktur unterbrechen würde. Unter weiterer Einbeziehung der Ergebnisse des Erfinders und anderer, daß es sich bei dem HIV-env-Glykoprotein um ein Tetramer handelt, kann man sich vorstellen, daß vier lange amphiphatische α-Helices eine umeinandergewundene Schraubenstruktur (coiled-coil) bilden, wobei jeweils die hydrophoben Regionen der Untereinheiten miteinander interagieren und dabei die oligomere Struktur von gp41 stabilisieren. In diesem Modell liegen die hydrophilen Aminosäureseitenketten außen an einer tetrameren Schraube, wobei bei verschiedenen Stämmen verschiedene Aminosäuren vorliegen können.

Aufbauend auf den vorgenannten Hypothesen wurde für den Einbau der Erkennungssequenz die Region zwischen der letzten Glykosylierungsstelle bei der Aminosäure 637 und dem Beginn der transmembranen Domäne ausgewählt, ein immerhin 40 bis 53 Aminosäuren langes Stück, je nachdem, wo man den Beginn des transmembranen Bereichs annimmt. Wesentlich für die Funktionalität der Erkennungssequenz ist, daß sie gut zugänglich sein muß für das entsprechende spaltende Enzym.

Auf der Basis dieser Erkenntnis, daß die Lokalisierung einer geeigneten Stelle für den Einbau einer Erkennungssequenz im wesentlichen von der Zugänglichkeit des entsprechenden Proteinbereichs für das spaltende Enzym abhängig ist, wurde ein neues Verfahren entwickelt zur Identifizierung einer geeigneten Einbaustelle für eine Erkennungssequenz, welches Gegenstand der Patentansprüche 4-7 ist.

Da Antikörper gegen ein Protein nur gegen diejenigen Strukturen gebildet werden können, die außen an dem Protein liegen, die also den Zellen des Immunsystems zugänglich sind, und die Zugänglichkeit auch ein wesentliches Kriterium für eine funktionsfähige Erkennungssequenz ist, können geeignete Bereiche im Protein für den Einbau von Erkennungssequenzen dadurch identifiziert werden, daß man diejenigen Epitope identifiziert, gegen die nach Immunisierung mit dem jeweiligen Protein Antikörper gebildet werden. Horal P. und Mitarbeiter haben zur Untersuchung, gegen welche Bereiche des HIV-1 env-Glykoproteins gp41 überhaupt Antikörper gebildet werden, die sogenannte "Pepscan"-Methode angewandt [siehe Horal P. et al. in der Zeitschrift "J. Virol.", **65**, 2718-2723 (1991)]. Diese Methode wird bei der vorliegenden Erfindung, anders als bei Horal et al. zur Identifikation des geeigneten Bereichs im Protein für die Erkennungssequenz eingesetzt. Hierzu werden im Prinzip kleine Bruchstücke des Proteins mit Antikörpern gemischt, die durch Immunisierung eines Säugetieres mit dem gesamten Protein gewonnen wurden. In der Praxis wird nicht das Protein in Bruchstücke zerlegt, sondern der Einfachheit halber entsprechend der Proteinsequenz überlappende Peptide mit einer Länge von 5 bis 30, insbesondere von 12 bis 16 Aminosäuren synthetisiert. Diese Peptide werden mit Serum von HIV-1-positiven Patienten versetzt. Die in diesem Serum vorhandenen Antikörper reagieren mit denjenigen Peptiden, die beim nativen Protein zugänglich an der Oberfläche liegen und die damit potentielle Einbaustellen für Erkennungssequenzen darstellen. Mit dieser Methode kann der am besten geeignete Abschnitt im Protein für den Einbau einer Erkennungssequenz je nach Lange der verwendeten Peptide bis auf einen Bereich weniger Aminosäuren identifiziert werden. Um die genaue, optimale Stelle zu finden, werden soviele Proteinmutanten hergestellt und getestet, wie nötig sind, um in dem zuvor identifizierten Bereich exakt die optimale Stelle für die Erkennungssequenz zu lokalisieren. Beim env-Glykoprotein von HIV-1 liegt die am besten geeignete Stelle für den Einbau einer Collagenasespaltstelle zwischen den Aminosäuren Leu 659 und Leu 660 (siehe Fig. 2). Aufgrund der Kolinearität von Gen- und Proteinsequenz entspricht dies den Nukleotiden A 7778 und T 7779 des env-Gens.
Ausgehend von dieser Stelle ist in einem Bereich von jeweils 17 Aminosäuren Richtung C-Terminus bzw. N-Terminus der Einbau einer Collagenasespaltstelle ebenfalls möglich, wobei mit zunehmendem Abstand mit geringeren Ausbeuten an Spattprodukten und damit der gewünschten Proteindomäne zu rechnen ist. Eine weitere Schwierigkeit stellte die Ermittlung der Struktur einer geeigneten Spaltstelle dar. Es gibt recht wenige Proteasen, die Polypeptide sequenzspezifisch spalten. Beispiele für hochspezifische Proteasen sind bakterielle, vereinzelt auch pflanzliche Collagenasen, oder der Blutgerinnungsfaktor Xa. Auch Erkennungssequenzen für Glycolipidanker sind prinzipiell geeignet. Erschwerend kommt hinzu, daß die meisten Endoproteasen, anders als beispielsweise DNA Restriktionsendonukleasen, für die Erkennung der Spaltstelle nicht nur auf die Sequenz, sondern auch auf bestimmte Sekundär- oder Tertiärstrukturen des Substrates angewiesen sind, welche aber nicht bekannt sind. Weiterhin darf die Erkennungssequenz nicht zu groß sein, um ausschließen zu können, daß durch ihre Größe die Struktur des Gesamtproteins wesentlich beeinflußt wird.

Am besten geeignet als Erkennungssequenz hat sich eine kurze collagenähnliche Sequenz der allgemeinen Form:

-(G - P - X)ₙ-

wobei n> 1, insbesondere 3 und X eine der 20 durch den genetischen Code festgelegten Aminosäuren ist (Anspruch 4).

Eine ähnliche Sequenz wird in der deutschen Patentanmeldung DE 37 31 874 vorgeschlagen, allerdings als Teil eines Fusionsproteins. Im Gegensatz zur vorliegenden Erfindung ist das Ziel, das in der Patentanmeldung DE 37 31 874 verfolgt wird, besonders kleine Proteine bzw. Peptide in Fermentationsverfahren herzustellen. Da aber gerade derartige Peptide verstärkt einem proteolytischen Abbau in der Zelle ausgesetzt sind, wird vorgeschlagen, das Peptid als Teil eines größeren Fusionsproteins zusammen mit einer bakteriellen Aminosäuresequenz und einer collagenähnlichen Sequenz in einer bakteriellen Wirtszelle zu synthetisieren und anschließend mit einer bakteriellen Collagenase zu spalten.

Unter Verwendung der genannten collagenähnlichen Sequenz wurden drei verschiedene HIV-1 env-Gen Mutanten (E3CA, E3CB, E3CC) hergestellt, deren Expressionsprodukt mittels einer bakteriellen Collagenase in der gewünschten Weise spezifisch gespalten werden kann. Unter Verwendung dieser Mutanten lassen sich Ektodomänen des env-Glykoproteins als wasserlösliche oligomere, insbesondere tetramere Glykoproteine gewinnen, deren Monomere eine elektrische Mobilität von ca. 140 kD aufweisen und daher gp140 genannt werden. Diese Proteine bestehen aus der Domäne gp120 und einem Teil der Domäne gp41 (Ansprüche 11 bis 14). Da sie keine Transmembrandomäne mehr aufweisen, sind anders als bei der Reinigung von komplettem gp160 keine starken Detergentien, welche die native Struktur der Glykoproteine verändern könnten, zur Aufreinigung dieser Glykoproteine notwendig. Klare Evidenzen dafür, daß gp140 oligomere Struktur besitzt, ergeben sich zum einen aus Sedimentationsanalysen [Fig. 4; siehe hierzu auch Earl, P.L. et al. "Proc. Natl. Acad. Sci. USA", **87**, 648-652 (1990)], zum anderen aus Crosslinking-Experimenten [zu dieser Methodik siehe Schawaller, M. et al., "Virology" **172**, 367-369 (1989)]. Die Gewinnung des oligomeren gp140 unter Verwendung der env-Gen Mutante E3CA wird im folgenden Ausführungsbeispiel in Verbindung mit den Figuren 1 bis 4 näher erläutert. Es zeigen:
- Fig. 1:: Schema des HIV-env-Glykoproteins mit der Transmembrandomäne in der Oberflächenmembran des Virus. Die Zahlen geben die Position der Aminosäuren an nach der Nomenklatur von Ratner et al., "Nature" **313**, 277-284 (1985). Die Domäne gp41 beginnt bei Aminosäure 512, die aber in einem solchen Schema schlecht lokalisiert werden kann.
- Fig.2:: Ausschnitte aus den Aminosäuresequenzen der Produkte von Wildtyp-env-Gen (E1(WT) des BH10-Klons und verschiedenen Mutanten. Die Veränderungen bei den Mutanten gegenüber dem Wildtyp-env-Produkt sind jeweils unterstrichen.

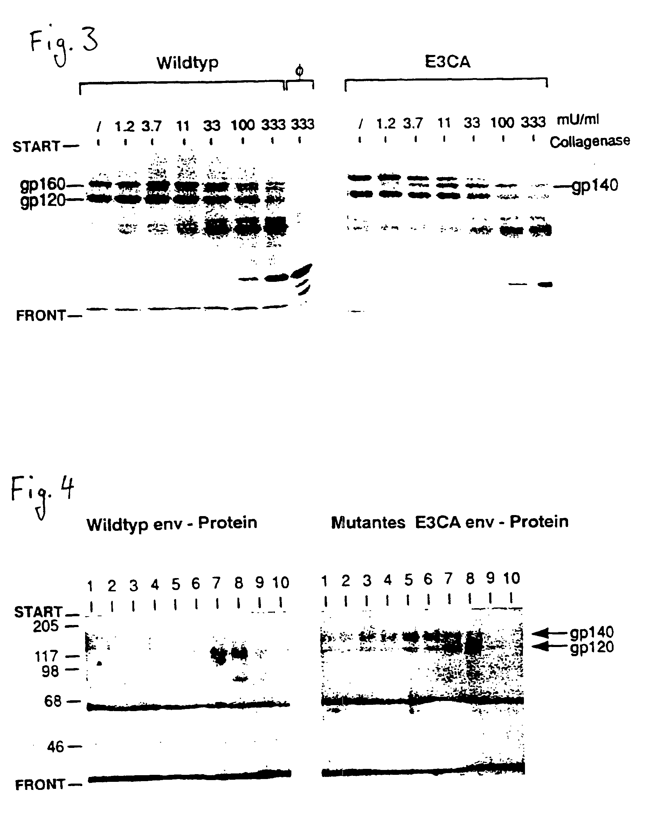

- Fig.3:: Spezifische Spaltung des Wildtyp-env- und des E3CA-env- Proteins durch unterschiedliche Konzentrationen an bakterieller Collagenase. Die erhaltenen Produkte wurden analysiert durch Immunoblotting nach gelelektrophoretischer Trennung auf 6% SDS Polyacrylamidgel und anschließende Detektion mit polyklonalem Kaninchenserum gegen gp120. Danach erfolgte eine Autoradiographie unter Einsatz eines radioaktiv markierten sekundären Antikörpers, der gegen Kaninchen-Immunoglobuline gerichtet ist (Amersham IM 134).
- Fig.4:: Sedimentationsanalyse der Produkte des Collagenaseverdaus von Wildtyp- und E3CA-Mutanten-Membranen. Collagenasekonzentration: 200 mU/ml, 37°C, 3h. Die erhaltenen Produkte wurden geschichtet auf einen vorgeformten 5 bis 20 % Sucrosegradienten in 10 mM HEPES, pH 7.8, 150 mM NaCl und in einem SW41 Rotor (Beckmann) für 16 h bei 35 000 UpM und 5°C zentrifugiert. Zehn Fraktionen wurden, vom Boden des Gefäßes angefangen, geerntet und durch Immunoblotting analysiert.

Die Konstruktion der env-Gen-Mutanten E3CA, E3CB, E3CC ist in Fig.2 illustriert, in der die Sequenz des Wildtyp env-Gens (E1) in der Transmembrandomäne und der aminoterminal zur Transmembrandomäne gelegenen Region dargestellt ist. An der für Leu 659 codierenden Position wurde durch eine Punktmutation eine einzelne Schnittstelle für die Endonuklease EcoR1 erzeugt, wodurch im Protein Leu gegen Phe ausgetauscht wird. Das daraus entstehende Produkt ist die env-Gen-Mutante E3. In diese neue EcoR1 Schnittstelle wurde eine Basensequenz insertiert, die für folgende Aminosäuresequenz codiert (zur Abkürzung von Aminosäuren siehe z.B. A.L. Lehninger, Biochemie, VCH-Verlag Weinheim, 1987):

V P A G P R G P R G P K F

Diese 13 Aminosäuren lange Sequenz stellt eine Collagenase Erkennungs- und Spaltstelle dar. Das Produkt mit diesem Insert wurde E3CA genannt. Das für diese Sequenz kodierende 39 Basenpaare lange Oligonukleotid wurde mit einem DNA - Synthesegerät der Firma Applied Biosystems synthetisiert. Zur Herstellung der env-Gen Mutanten sowie deren Klonierung in einen Expressionsvektor siehe auch T. Maniatis et al., Molecular Cloning, Cold Spring Harbor, N.Y., USA (1982).

### Konstruktion der env-Gen Mutanten

### a: Zerstörung der Prozessierungssequenz:

Zur Ausbeutensteigerung wurde zunächst die natürliche Prozessierungssequenz des env-Gen Produktes zerstört. In dem Plasmid pVB4 [Bosch, V. und Pawlita, M., "J. Virol. 64, 2337-2344 (1990)] wurde Adenosin 7334 gegen Cytosin ausgetauscht (A 7334 C), was beim Protein einen Austausch von Arginin 511 gegen Serin (Arg 511 Ser) bewirkt. Das so abgeänderte Plasmid pVB4 wurde danach kombiniert mit dem die Proteasespaltstelle enthaltenden E3CA-Plasmid (siehe unter c:). Hierzu wurde das 5'-Ende von pVB4 (Sall/HindIII-Fragment) mit dem 3'-Ende des E3CA-Plasmids (HindIII/Xhol-Fragment) ligiert und in den p-Bluescript Kloniert.

### b: Site-directed Mutagenese:

Gen von BH10 env (Sall/Xhol Fragment) wurde in einen M13 Vektor kloniert. Zum BH10-K,on siehe Ratner et al. "Nature" **313**, 277-284 (1985). Die Mutagenese zur Erzeugung der EcoR I - Erkennungssequenz erfolgte nach T Kunkel et al. ["Meth. Enzymol."154, 367-382 (1987)] mit dem Oligonukleotid MAN 59:

5` GAA CAA GAA TTC* TTG GAA TTA GAT AAA TGG 3`
659 660

Die mutagenisierte Base A 7781 C ist mit einem Stern markiert, die EcoR I-Erkennungsstelle ist fett markiert. Die Zahlen geben die Position der entsprechenden Aminosäuren im env-Gen Produkt an.

Die Verifizierung erfolgte mittels Sequenzierung. Die so erhaltene env-Gen Mutante wird bezeichnet als E3 (BH10, A7781C).

### c: Insertion von komplementären Oligonukleotiden:

Die im Blueskript pks+ enthaltene EcoRI Erkennungssequenz wurde zerstört durch Öffnen mittels EcoRI, Auffüllen mittels DNA Polymerase I (Klenow Fragment) und Verbinden der Enden mittels Ligase. Danach wurde env E3 in Blueskript pks+ subkloniert. Dann wurde E3 mit EcoR I linearisiert und über ein Agarosegel gereinigt.
Nun erfolgte die Insertion der 39 Nukleotide langen für die Collagenasespaltstelle codierenden Sequenz in diese EcoR I Erkennungsstelle von E3:

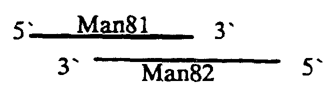

Die so erhaltene Mutante wird E3CA genannt. Zwei weitere Mutanten wurden erzeugt unter Verwendung der komplementären Oligonukleotidsequenzen Man83 und Man84 (E3CB) sowie Man85 und Man86 (E3CC). Diese Oligonukleotidsequenzen sind im folgenden dargestellt:

Man81 :

5`- A A T T A G T T C C T G C C G G T C C A A G A G G T C C A A G G G G A C C G A -3´

Man82:
5`- AATTTCGGTCCCCTTGGACCTCTTGGACCGGCAGGAACT -3`

Man83:
5`- AATTAGCCGTACCTGCCGGACCGCGTGGACCACGCGGACCTA -3`

Man84:

5`- A A T T T A G G T C C G C G T G G T C C A C G C G G T C C G G C A G G T A C G G C T -3`

Man85:
5`- AATTAGCAGCTGGTCCCGCAGGACCTCGCGGTCCTAGAGGTCCTA -3`

Man86:
5`- AATTTAGGACCTCTAGGACCGCGAGGTCCTGCGGGACCAGCTGCT -3`

Zur Insertion der komplementären Oligonukleotide in die EcoR I-Erkennungsstelle in E3 wurden die Oligonukleotide in 150 mM NaCl, TE (10mM Tris, 0,1mm EDTA. pH 8) gemischt (1:1, mol:mol). Anschließend wurde 2 h bei 95°C erhitzt und dann über 30 min auf Raumtemperatur abkühlen gelassen. Danach erfolgte die Ligase-Reaktion zusammen mit linearisiertem E3 (pks+) bei hohem Überschuß an dem jeweiligen Oligonukleotidpaar. Die so hergestellten Plasmide wurden in Echerichia coli DH5 transformiert und durch Sequenzierung analysiert. Die Ausbeute an neu rekombinanten Plasmiden beträgt über 90%.

### d: Klonierung in den Expressionsvektor pSVL:

Zur Expression der env-Gene wurden diese in den Expressionsvektor pSVL (Pharmacia, Uppsala, Schweden) kloniert (in pSVL wurde vorher die unique BamHI Erkennungsstelle deletiert). pSVL wurde mit Xhol linearisiert und die env-Gene als Sall/Xhol-Fragment (BH 10 Klon) subkloniert. Die env-Gene wurden unter die Kontrolle des SV40 Late Promotors gestellt.

Eine weitere Möglichkeit, einen die verschiedenen Gene enthaltenden Expressionsvektor herzustellen, besteht im sogenannten PCR (Polymerase Chain Reaction) -constructing. Eine Übersicht über diese Methodik ist zu finden bei Jones, D.H. et al, "Nature", **344,** 793-794 (1990).

Die env-Gen Mutanten E3CB und E3CC wurden aus folgendem Grund hergestellt: Es bestand durchaus die Möglichkeit, daß die Ausrichtung der Collagenasespaltstelle innerhalb der α-Helix Einfluß auf die Funktionsfähigkeit haben könnte. Da in einer α-Helix drei Aminosäuren ungefähr eine volle Umdrehung der Helix ausmachen, wurde durch Einfügen einer Aminosäure Alanin (E3CB) bzw. zweier Aminosäuren Alanin (E3CC) die Ausrichtung der Collagenasespaltstelle innerhalb der α-Helix um ca. 120° bzw. um ca. 240° verschoben. Die dadurch erhaltenen Mutanten E3CB und E3CC führten aber zum gleichen positiven Ergebnis wie die Mutante E3CA.

Die Expression der verschiedenen env-Gene wurde in COS 7-Zellen durchgeführt. COS 7-Zellen sind Fibroblasten, es können stattdessen aber im Prinzip alle eukaryontischen Zellen verwendet werden wie z.B. CV1-Zellen, Hefezellen, Insektenzellen [Summers, M.D. und Smith, G.E. : A Manual of Methods for Baculovirus Vectors and Insect Cell CuRure Procedures. Texas Agricultural Experiment Station; Bulletin No. 1555, (1988)] etc. Die COS 7-Zellen wurden mit den pSVL Plasmiden durch Elektroporation transformiert und anschließend für 48h in vollständigem Wachstumsmedium [Gluzman, Y. et al.: "Cell" **23**, 175-182 (1981)] inkubiert. Danach wurden die Zellen geerntet und die env-Proteine analysiert. Für die Transformation der Zellen mit dem Expressionsvektor können alle gängigen Verfahren angewandt werden, die Elektroporation ergibt aber im vorliegenden Fall besonders gute Erfolgsraten zwischen 10 und 50%.

### Elektroporation:

Ca. 10⁷ (2 x 10⁶ - 5 x 10⁷) COS 7 Zellen in logarithmischer Wachstumsphase wurden trypsiniert und mit DMEM (Dulbecco Modified Eagles' Medium) gewaschen. Zur Elektroporation wurden die Zellen mit 5 - 30 µg pSVL in DMEM versetzt und in einem BioRad Gene Pulser elektroporiert (Raumtemperatur; 800 µl; 250V; 500 µF; 0,4 cm Küvette).

Zur Analyse, ob die Collagenasespaltung zum gewünschten Erfolg führt, also ob nach Collagenasebehandlung der Mutante E3CA eine native Ektodomäne des env-Glykoproteins (gp140) extrahiert werden kann, wurden nach hypotonischer Zellyse mittels Sucrose-Dichtezentrifugation Membranpräparationen hergestellt [Schawaller, M. et al., "Virology" **172**, 367-369 (1989)].

### Reinigung der Membranpräparation:

Membranpräparation mehrfach waschen, in HEPES buffered saline (HBS: 10 mM HEPES 7,8; 150 mM NaCl) aufnehmen: + 1/10 Vol. 10 x HBS, + 1/100 Vol. 1 M CaCl₂, + 1/5 Vol. 20 % β-D-Octylglucosid bei 37°C. Danach Behandlung mit Potter. Mehrere Stunden bei 37°C inkubieren. Anschließend zentrifugieren für 30 min. bei 100 000 x g und 37°C. Der erhaltene Überstand wurde dann einem Proteaseverdau unterzogen mit gereinigter Collagenase (EC 3.4.24.3.) von *Clostridium histolyticum* (Sigma C0773, Deisenhofen) in Konzentrationen zwischen 1,2 und 333 mU/ml.

### Collagenaseverdau:

Der Collagenaseverdau wurde in folgendem Puffer für 3 h bei 37°C durchgeführt:
300 mM NaCI
20 mM HEPES (Hydroxyethylpiperazinethansulfonsäure), pH 7,8
10 mM CaCl₂
50µM ZnCl₂
4% β-D-Oktylglucosid

Diesem Puffer können folgende Proteaseinhibitoren zugefügt werden, um unspezifische Proteolyse zu minimieren:

| | |
|---|---|
| 1 µM E64 | (Cystein-Proteaseinhibitor) |
| 1 µM Pepstatin A | (Aspartyl-Proteaseinhibitor) |
| 1mM PMSF (Phenylmethylsulfonylfluorid) | (Serin-Proteaseinhibitor) |

Statt β-D-Oktylglucosid können auch andere milde Detergentien wie CHAPS oder DOC (Desoxycholat) verwendet werden. Zur Wirkung verschiedener Detergentien auf Membranproteine siehe auch Womack, M.D. et al. "Biochim. Biophys. Acta" **733**, 210-215 (1983).
Die optimale Collagenasekonzentration liegt zwischen 50 und 250 mU/ml (3h, 37°C). bei höheren Konzentrationen treten verstärkt unspezifische Verdauprodukte auf und die Ausbeute an gewünschtem Produkt, einem 140 kD Glykoprotein, wird geringer.

### Aufreinigung eines 140 kD env-Glykoproteins (gp140):

a: Lentil lectin-Affinitätschromatographie: Die proteolysierte Fraktion wurde 1:1 verdünnt mit destilliertem Wasser und aufgegeben auf eine Lentil Lectin Sepharose 4B - Säule (Pharmacia 17-0444-0'). Anschließend wurde gewaschen mit 5 Säulenvolumen HBS, 2% β-D-Oktylglucosid, danach mit 5 Säulenvolumen HBS und dann mit 5 Säulenvolumen 10 mM HEPES (pH 7,8), 50 mM NaCl. Anschließend wurde eluiert mit 500 - 1000 mM α-D-Methyl-Mannosid in 10 mM HEPES, 50 mM NaCl (evtl. erwärmen auf 37°C). Zur Lentil lectin-Affinitätschromatographie siehe auch Hayman, MJ. et al., "FEBS letters" **29** (2), 185-188 (1973).
   Das Eluat wurde anschließend aufkonzentriert. Gp140 liegt jetzt in einer Reinheit von ca. 50 - 70% vor.
b: Mono Q FPLC: Das Eluat aus a: wurde aufgenommen in 10 mM BTP (Bis-Trispropane), 50mM H₃BO₄, pH 8,2 und auf eine Mono Q HR 5/5 Säule (Pharmacia) aufgegeben, wobei die Proteinkonzentration bei etwa 1 mg/ml lag. Die Säule wurde eluiert mit einem NaCl-Gradienten von 30 - 600 mM über 40 min, 0,5 - 1 ml/min, UV-Monitor bei 280 nm. Analyse durch SDSPAGE Coomassie und/oder Western Blot. Das gp140 env-Protein eluiert bei ca. 400 mM NaCl. Gp140 liegt jetzt in einer Reinheit von ca. 80-90% vor. Die Zugabe von H₃BO₄ (Borat) zum Puffer bringt den Vorteil, daß dadurch das Aggregieren der Glykoproteine verhindert wird. Diese Verhinderung der Aggregation der Glykoproteine durch H₃BO₄ beruht wahrscheinlich auf einer Komplexierung der in Glykoproteinen häufigen vincinalen Hydroxylgruppen (Anspruch 8).
c: Sucrosedichtezentrifugation: Auf einen vorgeformten Sucrosegradienten 5-20% in HBS (10 mM HEPES, pH 7,8; 150 mM NaCI) wurde oben ca. 1 ml Eluat aus b: aufgeladen und in einem SW41-Rotor (Beckmann) zentrifugiert bei 35 000 UpM, 16h, 5°C. Anschließend Entnahme von 10 Fraktionen (von unten nach oben). In Fraktion 5 hat gp140 eine Reinheit von 90 - 95%. Analyse erfolgte durch SDS PAGE und/oder Western Blot. Da während der Zentrifugation keine Ca-lonen im Puffer enthalten sind, kann statt HEPES-Puffer beispielsweise auch Phosphatpuffer verwendet werden.

Die Proteolyseprodukte wurden analysiert mittels SDS-PAGE (Natriumlaurylsulfat-Polyacrylamidgelelektrophorese) und anschließendem Immunoblotting unter Verwendung eines polyklonalen Kaninchenserums, gerichtet gegen gp120, das sowohl gp120, als auch gp160 erkennt.

Der Immunoblot (Western-Blot) in Fig. 3 zeigt, daß das Wildtyp env-Gen (Klon BH10) und die Mutante E3CA etwa gleiche Mengen an gp160 exprimieren. Offensichtlich verläuft auch die proteolytische Prozessierung von gp160 zu gp120 und gp41, welche im Golgi-Apparat abläuft, bei Wildtyp-env- und Mutanten-env-Protein gleich, wenn die Prozessierungssequenz intakt ist. Nach Behandlung mit bakterieller Collagenase ergab das E3CA -env-Protein ein neues Verdauprodukt, dessen monomere Form eine elektrophoretische Mobilität von ca. 140 kD aufweist, welches beim Verdau des Wildtyp-env-Proteins nicht gefunden wurde. Dieses Verdauprodukt ist die gewünschte native Ektodomäne des env-Glykoproteins. Die mit 140 kD etwas geringere elektrophoretische Mobilität des neuen Verdauprodukts als die von gp120 ergibt sich daraus, daß die Collagenasespaltstelle bei Aminosäure 659 des Gesamtproteins gp160 im Bereich der Domäne gp41 eingebaut wurde. Die Domäne gp120 endet bei Aminosäure Arginin 511 (siehe auch Fig. 1). Die neu hergestellte native Ektodomäne des HIV-1 env-Glykoproteins, gp140, besteht also aus der gesamten Domäne gp120, einem 148 Aminosäuren langen Teilstück der Domäne gp41 sowie dem nach Collagenaseverdau verbleibenden Stück der collagenähnlichen Sequenz. Dieses letztgenannte Stück, das das C-terminale Ende des Glykoproteins bildet, ist zwar eine Art Fremdkörper in dem erfindungsgemäßen Glykoprotein, stört aber dessen Funktion nicht. Es kann, braucht aber nicht unbedingt entfernt zu werden.
Die elektrophoretische Mobilität von gp120 vom Wildtyp-env- und vom E3CA-env-Protein wird durch die Collagenasebehandlung nicht verändert. Das zeigt, daß die in der Mutante E3CA insertierte Collagenasespaltstelle von der bakteriellen Collagenase spezifisch erkannt und gespalten wird.

Ein weiterer Nachweis dafür, daß die in der Mutante E3CA insertierte Collagenasespaltstelle von der bakteriellen Collagenase spezifisch erkannt und gespalten wird, ergibt sich aus den Ergebnissen der Sedimentationsanalyse der Collagenasespaltprodukte (Fig. 4). Membranpräparationen von COS 7-Zellen, die das Wildtyp- oder das E3CA-env-Gen exprimieren, wurden mit 4% β-D-Oktylglycosid extrahiert und wie oben beschrieben mit Collagenase verdaut. Die erhaltenen Verdauprodukte wurden mittels Sucrose-Dichtezentrifugation über einen vorgeformten 5% - 20% Sucrosegradienten in 10 mM HEPES, pH 7.8, 150 mM NaCl in Abwesenheit von Detergentien zentrifugiert für 16 h bei 35 000 UpM in einem SW41 Rotor (Beckmann). Danach wurden insgesamt zehn Fraktionen entnommen und mittels SDS-PAGE und Immunoblotting auf ihren Gehalt an env-Proteinen untersucht. gp120 wurde in beiden Fällen in den Fraktionen 7 und 8 wiedergefunden, was dem monomeren gp120 mit einem Sedimentationskoeffizienten von 7S entspricht. Das Verdauprodukt von 140kD, das nur bei der E3CA-Mutante und nicht beim Wildtyp auftrat, wurde dagegen in Fraktion 5 wiedergefunden, was einem Sedimentatiortskoeffizienten von 11S entspricht.

In einer bevorzugten Ausführungsform wird ein rekombinantes Vaccinia Virus (VV) als Expressionsvektor verwendet. Die Konstruktion dieses rekombinanten Vaccinia Virus wird im folgenden Ausführungsbeispiel näher beschrieben.

Als Ausgangsmaterial wurde das Vaccinia shuttle plasmid p2100 verwendet [Patel, D. D. et al., "Proc. Natl. Acad. Sci. USA" 85, 9431-9435 (1988)]. Dieses enthält neben bakteriellem Origin und Resistenzgen folgendes Genfragment: 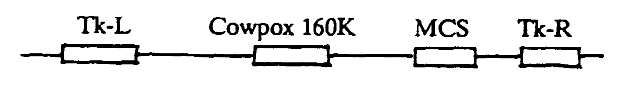
Tk-L Cowpox 160K MCS Tk-R
Tk-L = Linker Arm des Thymidinkinase-Gens
Tk-R = Rechter Arm des Thymidinkinase-Gens
Cowpox 160K = Promotor/Enhancer Sequenzen
MCS = Multiple cloning site

Durch Klonieren von doppelsträngigen Oligonukleotiden wurde 3' zum Promotor eine Lac O Sequenz, 24 bp palindrom, insertiert und die MCS um mehrere unique Endonuklease-Erkennungsstellen erweitert. Die Lac O Sequenz ist nötig, um die Transkription in E. coli auf ein Minimum zu unterdrücken.

In die neuen Restriktionsstellen wurde das jeweilige env-Gen (Wildtyp oder Mutante: aus pks Plasmid) cloniert (z. B. E3CA) [zur Methodik siehe Chakrabarti, S. et al., "Molec. Cell Biol." **5**, 3403-3409 (1985)].

Man erhält folgendes Konstrukt:

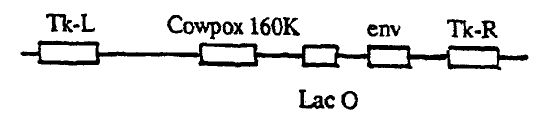
Tk-L Cowpox 160K env Tk-R
Lac O

Für die Herstellung rekombinanter Vaccinia Viren wurden die Plasmide analysiert durch Restriktionsanalyse und partielle Sequenzierung. Die Plasmide wurden in CV 1 Zellen transfiziert durch Elektroporation (Bedingungen wie bei COS-7 Zellen, siehe erstes Ausführungsbeispiel) und mit Wildtyp VV (Stamm Copenhagen) infiziert ( Muitiplicity of Infection (M O I) < 1). Es entstehen durch homologe Rekombination im Thymidinkinase-Gen des Plasmids mit dem Thymidinkinase-Gen des VV mit einer Wahrscheinlichkeit von etwa 1/1000 -1/10000 rekombinante VV, durch die das env-Gen exprimiert werden kann. Da diese phänotypisch TK⁻ sind, können sie durch 5'-Bromodeoxyuddin (BdUR)-Selektion in HTK⁻ Zellen positiv selektioniert werden. Zwei Runden BdUR/HTK- Selektion und anschließendes "Plaquen" (identifizieren von Plaques und entnehmen von rekombinanten VV) aus CV 1 Zellen ergibt ca. 50-90% env exprimierende Plaques (VV env), die durch Western Blot einer 1 ml Kultur identifiziert werden können. Nochmaliges Plaquen führt zu dem gewünschten rekombinanten Vaccinia Virus, durch den env exprimiert werden kann.

### Verwendung der viralen Membranproteindomänen:

Die vorstehend definierten erfindungsgemäßen nativen Domänen viraler Membranproteine können als Impfstoff gegen HIV eingesetzt werden. Hierzu werden sie dem Patienten vorzugsweise in Kombination mit geeigneten Adjuvantien verabreicht. Vorher müssen allerdings eventuell noch enthaltene Pufferbestandteile, wie beispielsweise HEPES, entfernt werden. Dies kann erfolgen z.B. durch Dialyse gegen physiologische Kochsalzlösung in Phosphatpuffer.

Weiterhin können die nativen Domänen der viralen Membranproteine zur Immuntherapie bei HIV-positiven Patienten verwendet werden.

Auch für diagnostische Zwecke, insbesondere zur Diagnose entsprechender Infektionen mit HIV sind die nativen viralen Proteindomänen geeignet. Zum einen können sie als Antigen beispielsweise in Immunoassays zum Nachweis spezifischer Antikörper dienen, zum anderen können sie auch eingesetzt werden zur Gewinnung von Antikörpern, sowohl monoklonalen als auch polyklonalen Antikörpern, die dann wiederum zum direkten Nachweis des Virus dienen können.

Ein weiterer wichtiger Verwendungszweck der aufgereinigten nativen Domänen viraler Membranproteine ist ihre Verwendung zur Kristallisation. Ist das Protein in kristalliner Form vorhanden, kann die dreidimensionale Struktur ermittelt werden. Dies ist insbesondere dahingehend interessant, als dadurch möglich wird, die genaue Struktur der Bindungstasche aufzuklären. Kennt man diese Struktur, ist es eventuell möglich, Inhibitoren zu konstruieren, das heißt, kleinere Moleküle, die genau in diese Bindungstasche hineinpassen und damit die Bindung des Proteins, also des Virus, an das CD4-Molekül, den Rezeptor der Wirtszelle, zu blockieren.

## Patentansprüche

1. Verfahren zur Gewinnung von isolierten Ektodomänen viraler Membranproteine in nativer, oligomerer und glykosylierter Form, dadurch gekennzeichnet, daß
- in das Gen, das für ein virales, die gewünschte Ektodomäne enthaltendes Membranprotein kodiert, eine Nukleotidsequenz insertiert wird, die für eine Aminosäuresequenz kodiert, welche eine Erkennungssequenz für proteinspaltende Enzyme darstellt,
- die so erhaltene Gen-Mutante in eukaryontischen Zellen zum Erhalt des Membranproteins in nativer, oligomerer und glykosylierter Form einschließlich der insertierten Erkennungssequenz exprimiert wird,
- die Ektodomäne in nativer, oligomerer und glykosylierter Form durch enzymatische Spaltung des Membranproteins mit für die Erkennungssequenz spezifischen proteinspaltenden Enyzmen von den eukaryontischen Zellen oder Teilen hiervon abgetrennt wird, und
- die Ektodomäne in nativer, oligomerer und glykosylierter Form aus dem Gemisch isoliert wird.

2. Verfahren nach Anspruch 1, wobei eine natürliche Prozessierungssequenz des Vorläuferproteins des Membranproteins durch Austausch mindestens einer Aminosäure zerstört wird.

3. Verfahren nach Anspruch 1 oder 2, wobei die Erkennungssequenz von Proteasen oder von Lipasen über Glycolipidankern erkannt und gespalten wird.

4. Verfahren nach einem der Ansprüche 1 bis 3, wobei die zu gewinnenden Ektodomänen die Ektodomänen des env-Glykoproteins von HIV sind.

5. Verfahren nach Anspruch 4, wobei die Nukleotidsequenz in einen Bereich des für das env-Glykoprotein kodierenden Gens insertiert wird, der bei dem daraus exprimierten env-Glykoprotein dem Bereich entspricht, der begrenzt wird durch die erste und fünfzigste Aminosäure vor Beginn des transmembranen Bereichs des env-Glykoproteins.

6. Verfahren nach Anspruch 4 oder 5, wobei die Erkennungssequenz in einen Bereich des env-Glykoproteins von HIV eingebaut wird, der durch die Aminosäuren 645 und 673 begrenzt wird.

7. Verfahren nach Anspruch 6, wobei der Bereich durch die Aminosäuren 656 und 663 begrenzt wird.

8. Verfahren nach einem der Ansprüche 4 bis 7, wobei die Erkennungssequenz zwischen den Aminosäuren 659 und 660 in das env-Glykoprotein von HIV eingebaut wird.

9. Verfahren nach einem der Ansprüche 1 bis 8, wobei
die Erkennungssequenz die folgende allgemeine Form besitzt:
-(G - P - X)ₙ -
wobei n eine ganze Zahl größer 1, insbesondere 3, und X eine der 20 durch den genetischen Code festgelegten Aminosäuren ist, und
das für die Spaltung verwendete Enzym eine Collagenase ist.

10. Verfahren nach Anspruch 9, wobei die Collagenase eine bakterielle Collagenase ist.

11. Verfahren nach einem der Ansprüche 1 bis 10, worin der Puffer, der bei der Reinigung der gewünschten Ektodomäne aus dem Gemisch nach der enzymatischen Spaltung verwendet wird, Borat in einer Konzentration zwischen 1 und 200 mM enthält.

12. Verfahren nach Anspruch 11, wobei der Puffer Borat in einer Konzentration zwischen 30 und 100 mM enthält.

13. Verfahren nach einem der Ansprüche 1 bis 12, worin ein für den Einbau einer Erkennungssequenz in Membranproteine geeigneter Bereich dadurch identifiziert wird, daß entsprechend der Sequenz des untersuchten Membranproteins synthetisierte, kurze, überlappende Peptide mit Serum eines gegen dieses Membranprotein immunisierten Säugetieres gemischt werden, wobei diejenigen Peptide, die mit den in diesem Serum enthaltenen Antikörpern reagieren, für den Einbau einer Erkennungssequenz geeignete Bereiche in dem untersuchten Membranprotein darstellen.

14. Verfahren nach Anspruch 13, wobei die synthetisierten, kurzen, überlappenden Peptide mit Serum HIV positiver Menschen gemischt werden.

15. Verfahren nach Anspruch 13 oder 14, wobei die synthetisierten Peptide eine Länge zwischen 5 und 30 Aminosäuren, insbesondere zwischen 12 und 16 Aminosäuren, aufweisen.

16. Verfahren nach Anspruch 15, wobei die synthetisierten Peptide eine Länge zwischen 12 und 16 Aminosäuren aufweisen.

17. Isolierte Ektodomänen viraler Membranproteine, dadurch gekennzeichnet, daß sie in nativer, oligomerer und glykosylierter Form vorliegen, wobei das korrespondierende Gen des viralen Membranproteins derart manipuliert ist, daß das virale Membranprotein eine zusätzliche Aminosäuresequenz beinhaltet, die als Erkennungssequenz für proteinspaltende Enzyme oder für Glykolipidanker dient, wobei die isolierten Ektodomänen aus der Domäne gp120 des HIV env-Glykoproteins, dem 131 bis 165 Aminosäuren langen aminoterminalen Stück der Domäne gp41 des HIV env-Glykoproteins sowie gegebenenfalls dem nach der enzymatischen Spaltung verbleibenden Stück der Erkennungssequenz gemäß Anspruch 5 zusammengesetzt sind.

18. Isolierte Ektodomänen nach Anspruch 17, wobei sie in tetramerer Form vorliegen.

19. Isolierte Ektodomänen nach einem der Ansprüche 17 oder 18, wobei ihre monomere Form eine elektrophoretische Mobilität von etwa 140 kD besitzt.

20. Isolierte Ektodomänen nach einem der Ansprüche 17 bis 19, wobei das aminoterminalen Stück der Domäne gp41 des HIV env-Glykoproteins 148 Aminosäuren lang ist.

21. Pharmazeutische Zusammensetzung, enthaltend isolierte Ektodomänen viraler Membranproteine nach einem der Ansprüche 17 bis 20.

22. Pharmazeutische Zusammensetzung nach Anspruch 21 als Impfstoff gegen HIV, oder zur Immuntherapie bei HIV-positiven Patienten.

23. Diagnostisches Mittel, enthaltend isolierte Ektodomänen viraler Membranproteine nach einem der Ansprüche 17 bis 20.

24. Diagnostisches Mittel nach Anspruch 23, zur Diagnose entsprechender Infektionen mit HIV.

25. Verwendung isolierter Ektodomänen viraler Membranproteine nach einem der Ansprüche 17 bis 20 für die Gewinnung von polyklonalen oder monoklonalen Antikörpern, die gegen diese Proteindomänen gerichtet sind.

26. Verwendung isolierter Ektodomänen viraler Membranproteine nach einem der Ansprüche 17 bis 20 für die Kristallisation dieser Proteindomänen.

## Revendications

1. Procédé d'obtention d'ectodomaines isolés de protéines virales membranaires, sous forme native, oligomère et glycosylée, caractérisé en ce que :
- dans le gène qui code pour une protéine membranaire virale contenant l'ectodomaine concerné, on insère une séquence nucléotidique qui code pour une séquence d'acides aminés qui forme une séquence de reconnaissance pour une enzyme de coupure de protéine,
- le gène mutant ainsi obtenu est exprimé dans des cellules eucaryotes pour l'obtention de la protéine membranaire sous forme native, oligomère et glycosylée incluant la séquence de reconnaissance insérée,
- l'ectodomaine, sous forme native, oligomère et glycosylée, est séparé des cellules eucaryotes ou des parties de celles-ci par coupure enzymatique de la protéine membranaire à l'aide d'une enzyme de coupure de protéine, spécifique de la séquence de reconnaissance,
- l'ectodomaine, sous forme native, oligomère et glycosylée, est isolé du mélange.

2. Procédé selon la revendication 1, dans lequel une séquence naturelle de traitement de la protéine précurseur de la protéine membranaire est dégradée par substitution d'au moins un acide aminé.

3. Procédé selon la revendication 1 ou 2, dans lequel la séquence de reconnaissance d'une protéase ou d'une lipase est reconnue et coupée par l'intermédiaire d'un glycolipide.

4. Procédé selon l'une quelconque des revendications 1 à 3, dans lequel les ectodomaines à obtenir sont des ectodomaines de glycoprotéines-env de VIH.

5. Procédé selon la revendication 4, dans lequel la séquence de nucléotides est insérée dans un domaine du gène codant pour la glycoprotéine-env, qui correspond au domaine par lequel est exprimé la glycoprotéine-env, qui est limité par le premier et le cinquantième acide aminé avant le début du domaine transmembranaire de la glycoprotéine-env.

6. Procédé selon l'une quelconque des revendications 4 ou 5, dans lequel la séquence de reconnaissance est insérée dans un domaine de la glycoprotéine-env du VIH limité par les acides aminés 645 et 673.

7. Procédé selon la revendication 6, dans lequel le domaine est limité par les acides aminés 656 et 663.

8. Procédé selon l'une quelconque des revendications 4 à 7, dans lequel la séquence de reconnaissance est insérée dans la glycoprotéine-env du VIH entre les acides aminés 659 et 660.

9. Procédé selon l'une quelconque des revendications 1 à 8, dans lequel :
- la séquence de reconnaissance a la forme générale suivante :
-(G-P-X)ₙ-
dans laquelle
- n est un nombre entier supérieur à 1, de préférence 3 et X est un des 20 acides aminés définis par le code génétique ; et
- l'enzyme utilisée pour la coupure est une collagénase.

10. Procédé selon la revendication 9 dans lequel la collagénase est une collagénase bactérienne.

11. Procédé selon l'une quelconque des revendications 1 à 10, dans lequel le tampon employé pour la purification de l'ectodomaine recherché du mélange après la coupure enzymatique contient du borate à une concentration 1 à 200 mM.

12. Procédé selon la revendication 11, dans lequel le tampon contient 30 à 100 mM de borate.

13. Procédé selon l'une quelconque des revendications 1 à 12, dans lequel un domaine convenant à l'insertion d'une séquence de reconnaissance dans une protéine membranaire est identifié par mélange de peptides courts chevauchants synthétisés, correspondant à la séquence de la protéine membranaire examinée avec un sérum de mammifère immunisé contre cette protéine membranaire, dans lequel les peptides qui réagissent avec les anticorps contenus dans ce sérum forment, dans la protéine membranaire examinée, un domaine convenant à l'insertion d'une séquence de reconnaissance.

14. Procédé selon la revendication 13 dans lequel les peptides courts, chevauchants, synthétisés sont mélangés avec du sérum d'un être humain positif pour le VIH.

15. Procédé selon l'une quelconque des revendications 13 ou 14, dans lequel les peptides synthétisés présentent une longueur de 5 à 30 acides aminés, en particulier de 12 à 16 acides aminés.

16. Procédé selon la revendication 15, dans lequel les peptides synthétisés ont une longueur de 12 à 16 acides aminés.

17. Ectodomaines isolés des protéines membranaires virales, caractérisés en ce qu'ils se trouvent sous forme native, oligomère et glycosylée, le gène correspondant à la protéine membranaire virale étant manipulé de telle sorte que la protéine membranaire contienne une séquence d'acides aminés supplémentaire qui sert de séquence de reconnaissance pour une enzyme coupée par une protéine ou pour un glycolipide, les ectodomaines isolés étant constitués du domaine gp120 de la glycoprotéine-env de VIH, du segment N-terminal ayant une longueur de 131 à 165 acides aminés du domaine gp41 de la glycoprotéine-en de VIH, ainsi qu'éventuellement du segment restant après coupure enzymatique de la séquence de reconnaissance selon la revendication 5.

18. Ectodomaines isolés selon la revendication 17, dans lesquels ceux-ci se trouvent sous forme de tétramère.

19. Ectodomaines isolés selon l'une quelconque des revendications 17 ou 18, dans lequel sa forme monomère présente une mobilité électrophorétique d'environ 140 kD.

20. Ectodomaines isolés selon l'une des revendications 17 à 19, dans lequel le segment N-terminal du domaine gp41 de la glycoprotéine-env du VIH a une longueur de 148 acides aminés.

21. Composition pharmaceutique contenant des ectodomaines isolés de protéines membranaires virales selon l'une quelconque des revendications 17 à 20.

22. Composition pharmaceutique selon la revendication 21 en tant que vaccin antiviral, en particulier contre le VIH ou pour une immunothérapie, chez des patients positifs pour le VIH.

23. Moyen de diagnostic, comprenant des ectodomaines isolés de protéines membranaires virales selon l'une quelconque des revendications 17 à 20.

24. Moyen de diagnostic selon la revendication 23, pour le diagnostic des infections par le VIH.

25. Utilisation d'ectodomaines isolés de protéines membranaires virales selon l'une quelconque des revendications 17 à 20, pour l'obtention d'anticorps polyclonaux ou monoclonaux qui sont dirigés contre ces domaines de protéines.

26. Utilisation d'ectodomaines isolés de protéines membranaires virales selon l'une quelconque des revendications 17 à 20, pour la cristallisation de ces domaines de protéines.

## Claims

1. A method of obtaining isolated ectodomains of viral membrane proteins in native, oligomeric and glycosylated form, characterized in that
- a nucleotide sequence coding for an amino acid sequence which represents a recognition sequence for protein-cleaving enzymes is inserted into the gene coding for a viral membrane protein containing the desired ectodomain;
- the gene mutant obtained in this manner is expressed in eukaryotic cells for obtaining the membrane protein in native, oligomeric and glycosylated form including the inserted recognition sequence;
- the ectodomain is separated in native, oligomeric and glycosylated form by enzymatic cleavage of the membrane protein with protein-cleaving enzymes specific for the recognition sequence from the eukaryotic cells or parts thereof; and
- the ectodomain is isolated in native, oligomeric and glycosylated form from the mixture.

2. The method according to claim 1 in which a natural processing sequence of the precursor protein of the membrane protein is destroyed by the replacement of at least one amino acid.

3. The method according to claim 1 or 2 in which the recognition sequence is recognized and cleaved by proteases or by lipases via glycolipid anchors.

4. The method according to any one of claims 1 to 3 in which the ectodomains to be obtained are the ectodomains of the env glycoprotein of HIV.

5. The method according to claim 4 in which the nucleotide sequence is inserted into a region of the gene coding for the env glycoprotein which region corresponds in the case of the env glycoprotein expressed therefrom to the region limited by the first and the fiftieth amino acid before the beginning of the transmembrane region of the env glycoprotein.

6. The method according to claim 4 or 5 in which the recognition sequence is inserted into a region of the env glycoprotein of HIV which is limited by the amino acids 645 and 673.

7. The method according to claim 6 in which the region is limited by the amino acids 656 and 663.

8. The method according to any one of claims 4 to 7 in which the recognition sequence is inserted into the env glycoprotein of HIV between amino acids 659 and 660.

9. The method according to any one of claims 1 to 8 in which
- the recognition sequence has the following general form:
-(G - P - X)ₙ -
in which n is an integer of greater than 1, especially 3, and X is one of the 20 amino acids determined by the genetic code; and
- the enzyme used for the cleavage is a collagenase.

10. The method according to claim 9 in which the collagenase is a bacterial collagenase.

11. The method according to any one of claims 1 to 10 in which the buffer used in the purification of the desired ectodomain from the mixture after the enzymatic cleavage contains borate in a concentration between 1 and 200 mM.

12. The method according to claim 11 in which the buffer contains borate in a concentration between 30 and 100 mM.

13. The method according to any one of claims 1 to 12 in which a region suitable for the insertion of a recognition sequence into membrane proteins is identified in that short, overlapping peptides synthesized in accordance with the sequence of the membrane protein to be investigated are mixed with serum of a mammal immunized against this membrane protein, wherein those peptides which react with the antibodies contained in this serum represent regions in the membrane protein investigated which are suitable for the insertion of a recognition sequence.

14. The method according to claim 13 in which the synthesized, short, overlapping peptides are mixed with serum of HIV-positive humans.

15. The method according to claim 13 or 14 in which the synthesized peptides have a length between 5 and 30 amino acids, especially between 12 and 16 amino acids.

16. The method according to claim 15 in which the synthesized peptides have a length between 12 and 16 amino acids.

17. Isolated ectodomains of viral membrane proteins, characterized in that they are present in native, oligomeric and glycosylated form wherein the gene corresponding to the viral membrane protein is manipulated such that the viral membrane protein includes an additional amino acid sequence which serves as a recognition sequence for protein-cleaving enzymes or for glycolipid anchors, wherein the isolated ectodomains are composed of domain gp120 of the HIV env glycoprotein, the 131 to 165 amino acids long amino-terminal section of domain gp41 of the HIV env glycoprotein as well as optionally the section of the recognition sequence according to claim 5 remaining after the enzymatic cleavage.

18. The isolated ectodomains according to claim 17 which are present in tetrameric form.

19. The isolated ectodomains according to claim 17 or 18 whose monomeric form has an electrophoretic mobility of approximately 140 kD.

20. The isolated ectodomains according to any one of claims 17 to 19 in which the amino-terminal section of domain gp41 of the HIV env glycoprotein is 148 amino acids long.

21. A pharmaceutical composition containing isolated ectodomains of viral membrane proteins according to any one of claims 17 to 20.

22. The pharmaceutical composition according to claim 21 as vaccine against HIV, or for immunotherapy in the case of HIV-positive patients.

23. A diagnostic agent containing isolated ectodomains of viral membrane proteins according to any one of claims 17 to 20.

24. The diagnostic agent according to claim 23 for diagnosing corresponding infections with HIV.

25. The use of isolated ectodomains of viral membrane proteins according to any one of claims 17 to 20 for the obtention of polyclonal or monoclonal antibodies directed against these protein domains.

26. The use of isolated ectodomains of viral membrane proteins according to any one of claims 17 to 20 for the crystallization of these protein domains.
